# EUROPÄISCHE PATENTANMELDUNG

(11) **EP 2 368 984 A1**
(43) Veröffentlichungstag der Anmeldung: **28.09.2011**
(21) Anmeldenummer: 10011604.5
(22) Anmeldetag: 13.09.2004
(51) Int. Cl.: C12N 9/72, A61K 38/49

(54) **Plasminogen-Aktivatoren mit verringerter Lysin-Bindungskapazität**

(30) Priorität: 12.09.2003 DE 10342518
(62) Teilanmeldung aus: 04765139.3
(71) Anmelder: Paion Deutschland GmbH, 52062 Aachen (DE)
(72) Erfinder: Söhngen, Wolfgang, 52080 Aachen (DE); Kops, Oliver, 52078 Aachen (DE); Ellis, Vincent, Norwich NR 4779 (GB)
(74) Vertreter: Von Renesse, Dorothea

(57) **Zusammenfassung**

Verwendung von Plasminogen-Aktivatoren mit verringerter Lysin-Bindungskapazität zur Behandlung thrombotischer Erkrankung.

## Beschreibung

Die Erfindung betrifft eine vorteilhafte Verwendung von Plasminogen-Aktivatoren und nimmt die Priorität der deutschen Patentanmeldung 103 42 518.7 in Anspruch, auf die inhaltlich Bezug genommen wird.

Die Etablierung der Thrombolyse als Therapieoption bei unterschiedlichen thromboembolischen Erkrankungen gilt als weitgehend abgeschlossen. Das Hauptinteresse der Thrombolyseforschung gilt daher der Weiterentwicklung und Modifikation bekannter Thrombolytika und/oder der Verbesserung der Begleittherapien.

Ein wichtiges Thrombolytikum als Ausgangspunkt neuer Thrombolytika ist der Gewebe-Plasminogen-Aktivator (t-PA), der im Vergleich zu Streptokinase oder Urokinase eine erhöhte Fibrinselektivität und eine verbesserte Wirksamkeit aufweist. Weiterentwicklungen dieses Plasminogen-Aktivators stellen die Deletionsmutanten Reteplase und Lanoteplase sowie die Tenecteplase dar.

Der Gewebe-Plasminogen-Aktivator (rt-PA) ist ein einkettiges Glykoprotein aus 527 Aminosäuren. Das zunächst einkettige Molekül (single-chain; sct-PA) wird durch Proteolyse in eine zweikettige Form (two chain; tct-PA) gespalten. Das t-PA weist bestimmte strukturelle und funktionelle Domainen auf. So umfaßt die N-terminale Teilkette die sog. Fingerdomaine (F, Ser1-Lys49), die epidermale Wachstumsfaktordomaine (E, Ser50 - Asp87) sowie die zwei Kringeldomainen (K1, Thr88 - Gly176; und K2, Asn177 - Cys261). Die C-terminale Kette mit einer Serinproteinasedomaine (P) umfaßt die Aminosäuren Ser262 bis Pro527. Die C-terminale Kette bildet mit den Aminosäuren His322, Asp371 und Ser478 das aktive Zentrum.

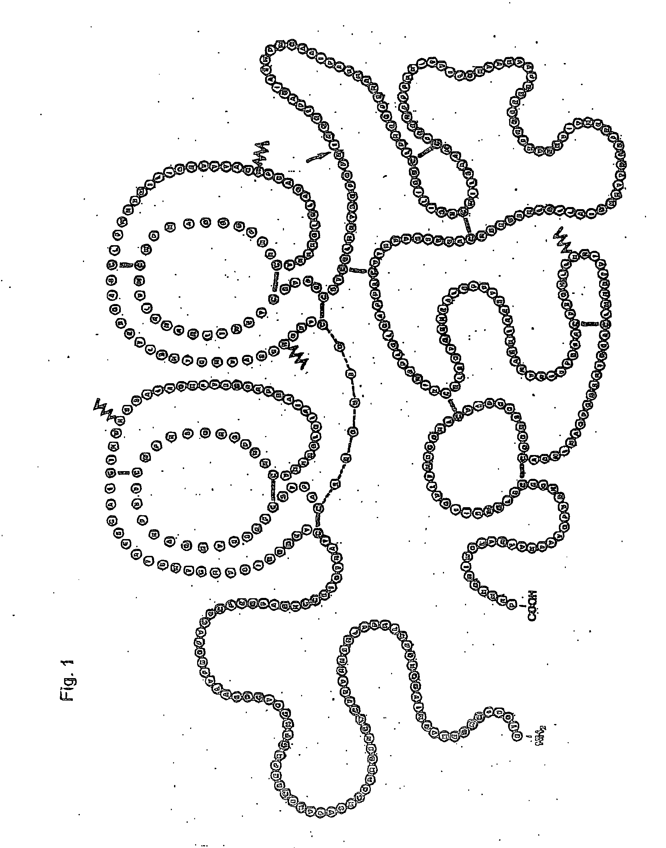

Das t-PA sowie dessen rekombinante Varianten und deren Herstellung sind Gegenstand beispielsweise des US-Patentes 4,766,075 und vielfältiger Publikationen (so z.B. Bode und Renatus: Tissue-type plasminogen activator: variants and crystal/solution structures demarcate structural determinants of function, Current Opinion in Structural Biology 1997, 7: 865 - 872). Eine schematische Darstellung der t-PA-Struktur ist als Fig. 1 beigefügt.

Wie die meisten anderen Trypsin-ähnlichen Serinproteinasen wird das sct-PA durch Spaltung in die tct-PA-Form überführt. Die Spaltung erfolgt an der Bindung zwischen Arg275 und Ile276. Danach werden die zwei Ketten nur noch über eine einzige Disulfidbrücke zwischen Serin264 und Serin395 zusammengehalten.

Diese Numerierung der Spaltstelle des t-PA entspricht derjenigen, die Bode und Renatus (a.a.O) gewählt haben. Andere Autoren legen jedoch eine andere Numerierung zugrunde und definieren die Spalt- oder Aktivierungsstelle als R15-I16 oder R310-I311. Funktionell und strukturell unterscheiden sich die so definierten Stellen aber nicht.

Der Gewebe-Plasminogen-Aktivator (t-PA; Alteplase) kann Plasminogen zu Plasmin aktivieren. Aus kinetischen Konstanten geht hervor, daß t-PA zirkulierendes Plasminogen jedoch nur mit schwacher Aktivität aktiviert. Beide t-PA-Formen - d.h. sowohl das einkettige als auch das zweikettige Molekül - weisen prinzipiell die gleichen pharmakologischen Eigenschaften auf. Das an Fibrin gebundene Plasminogen wird gegenüber dem freien Plasminogen jedoch mit einer um drei Zehnerpotenzen verbesserten katalytischen Potenz aktiviert. Demnach wird die thrombolytische Eigenschaft des Gewebe-Plasminogen-Aktivators durch die Anwesenhelt von Fibrin außerordentlich verstärkt.

Es wird für t-PA eine relative Fibrinselektivität mit einem Faktor 500 bis 1000 angegeben. Eine Steigerung der katalytischen Wirkung kann auch durch die

Interaktion des Plasminogen-Aktivators mit β-Amyloid oder Fibrinogen erreicht werden. Die Aktivierbarkelt der t-PA durch Fibrinogen ist vergleichbar mit der Aktivierbarkeit durch β-Amyloid.

Die Wirkung des Plasminogen-Aktivators wird physiologisch durch Inhibitoren kontrolliert, wobei der Plasminogen-Aktivator-Inhibitor I (PAI-1) den wesentlichen Gegenspieler darstellt. Durch Bindung von PAI-1 an die leichte Teilkette des t-PA-Moleküls wird die Struktur des katalytischen Zentrums so verändert, daß keine Aktivierungsreaktion mit dem Plasminogen mehr erfolgen kann (Bennet WF, Paoni NF, Keyt BA, Botstein D, Jones AJS, Presta L, Wurm FM, Zoller M: High resolution analysis of functional determinants in human tissue-type plasminogen activator. Journal of Biological Chemistry 1991; 266: 5191-5201).

Der Gewebe-Plasminogen-Aktivator wird rasch über die Leber metabolisiert. Daher verlängert die Leberinsuffizienz eines Patienten die Plasma-Halbwertzeit der Substanz (Emeis JJ, van den Hoogen CM, Jense D: Hepatic clearance of tissue-type plasminogen activator in rats, Thomb Haemostas 1985; 54:661-664; Tiefenbrunn AJ, Robison AK. Kumik PB, Ludbrook PA, Sobel BE. Clinical pharmacology in patients with evolving myocardial infarction of tissue plasminogen activator produced by recombinant DNA technology Circulation 1985; 71: 110-116).

Plasminogen-Aktivatoren werden zur Behandlung thrombotischer Erkrankungen wie beispielsweise des Herzinfarktes und des Schlaganfalls entwickelt. Bei t-PA handelt es sich um das einzige derzeit für die Behandlung des Schlaganfalls von der Food and Drug Administration (FDA) in den USA zugelassene Thrombolytikum.

Gleichwohl mehren sich in der Vergangenheit die Vermutungen, daß t-PA bei der Behandlung des Schlaganfalls zwar einerseits die erwarteten positiven thrombolytischen Effekte zeigt, andererseits aber auch für eine ungewollte Gewebezerstörung verantwortlich ist. So verursachte die Infusion von t-PA in t-PA-defizienten Mäusen größere Infarkte (Wang YF, Tsirka SE. Strickland S, Stieg, PE, Soriano SG. Lipton SA: Tissue plasminogen activator (tPA) increases neuronal damage after focal cerebral ischemia in wildtype and tPA-deficient mice. Nat Med 1998; 4(2):228-231). Diese vergrößerten Läsionen sind - so wird vermutet - auf die Stimulierung der NMDA abhängigen Glutamat-Rezeptoren zurückzuführen (Liberatore GT. Samson A, Bladin C, Schleuning WD, Medcalf RL: Vampire bat salivary plasminogen activator (desmoteplase): a unique fibrinolytic enzyme that does not promote neurodegeneration. Stroke 2003;34(2):537-543). Für diesen Effekt könnte eine proteolytische Spaltung des NMDA-Rezeptors durch t-PA verantwortlich sein (Nicole O, Docagne F, Ali C, Margaill I, Carmeliet P, MacKenzie ET, et al: The proteolytic activity of tissue-plasminogen activator enhances NMDA receptor-mediated signaling. Nat Med 2001;7(1):59-64).

Es ist daher Aufgabe der vorliegenden Erfindung, eine neue therapeutische Behandlung thrombotischer Erkrankungen, insbesondere des Schlaganfalls, bereitzustellen.

Diese Aufgabe wird gelöst durch die Verwendung eines Plasminogen aktivierenden Faktors, der eine gegenüber dem nativen Plasminogen-Aktivator verringerte Kapazität zur Bindung von Lysin aufweist. In einer besonders vorteilhaften Ausführungsform weist der Plasminogen-Aktivator eine modifizierte Kringel-Domaine, vorzugsweise eine im Vergleich zum nativen t-PA modifizierte Kringel 2-Domaine auf. Diese kann vollständig oder in Teilen deletiert sein, so daß die Lysin-Bindungskapazität verringert ist.

Die K2-Domaine - oder dazu funktionell oder strukturell im wesentlichen homologe Bereiche - können vorteilhafterweise so modifiziert sein, daß die Lysinreste nicht mehr oder nur mit geringer Affinität binden.

In einer besonders vorteilhaften Ausführungsform handelt es sich bei dem erfindungsgemäß einsetzbaren Plasminogen-Aktivator um modifiziertes t-PA.

Die Bedeutung der K2-Domaine einschließlich der Herstellung von t-PA K2 Deletionsmutanten wird ausführlich beschrieben bei Horrevoets AJ, Smilde A, de Vries C, Pannekoek H (The specific roles of finger and kringle 2 domains of tissue-type plasminogen activator during in vitro fibrinolysis: Journal of Biological Chemistry, 269, 17, 12639-12644, 1994).

Die gemäß der Erfindung einsetzbaren Plasminogen aktivierenden Faktoren zeigen aufgrund ihrer fehlenden oder verminderten Lysin-Bindungsfähigkeit eine erhöhte Fibrinselektivität und eine verringerte Aktivierbarkeit durch Fibrinogen oder β-Amyloid. Diese reduzierte Fibrinogen-Aktivierbarkeit ist vermutlich die Ursache für die Fibrinselektivität. Insbesondere bei der Behandlung von Schlaganfall ist ein hohe Fibrinselektivität des Plasminogen-Aktivators von wesentlicher Bedeutung, da beispielsweise das native t-PA über das die beschädigte Blut-Hirnschranke überwindende Fibrinogen aktiviert werden kann und über die anschließende Aktivierung der NMDA-Rezeptoren die Glutamat vermittelte Exzitotoxizität stimuliert. Die erfindungsgemäße Verringerung der Fibrinogen-Aktivierbarkeit des Plasminogen-Aktivators führt demnach auch zu einer verringerten Neurotoxizität.

Die Bedeutung der Lysin-Bindungsstellen am Kringel-2 des t-PAs wurde vor allem durch vergleichende Untersuchungen der strukturellen und funktionellen Domainen des t-PA im Vergleich zum DSPA deutlich. Bei DSPA handelt es sich um einen Plasminogen-Aktivator, der ursprünglich aus dem Speichel der Vampirfledermaus isoliert wurde (siehe dazu US-Patent 6,008,019; EP 0 383 417). Das DSPA ist in vier Isoformen isolierbar, von der das DSPAα1 rekombinant über CHO-Zellen herstellbar ist.

DSPA weist im Vergleich zum t-PA lediglich eine Kringel-Domaine auf. Diese Domaine entspricht funktionell und strukturell eher der K1-Domaine des t-PA als der K2-Domaine und weist keine Lysin-Bindungsstellen auf (Bringmann P, Gruber D. Liese A, Toschi L, Krätzschmar J, Schleuning WD, Donner P: Structural features mediating fibrin selectivity of vampire bat plasminogen activators; Journal of Biological Chemistry 1995;270(43):25596-25603). Es wird daher in der Literatur zum Teil davon gesprochen, dass DSPA keine Kringel 2 Domaine aufweist.

Des weiteren liegt DSPA stets als einkettiges Molekül vor, da eine Plasminaktivierungsstelle wie im t-PA fehlt. DSPA zeigt im Vergleich zu t-PA eine Stimulierung der Aktivität in Gegenwart von Fibrin um das 45000-fache, während dieser Wert nach Gardell SJ, Duong LT. Diehl, York JD, Hare TR, Register RB, Jacobs JW. Dixon RA, Friedman PA (Isolation, characterization and c-DNA cloning of a vampire bat salivary plasminogen activator: Journal of Biological Chemistry 1989;264(30):17947-17952) bei einem Wert von 205 liegt.

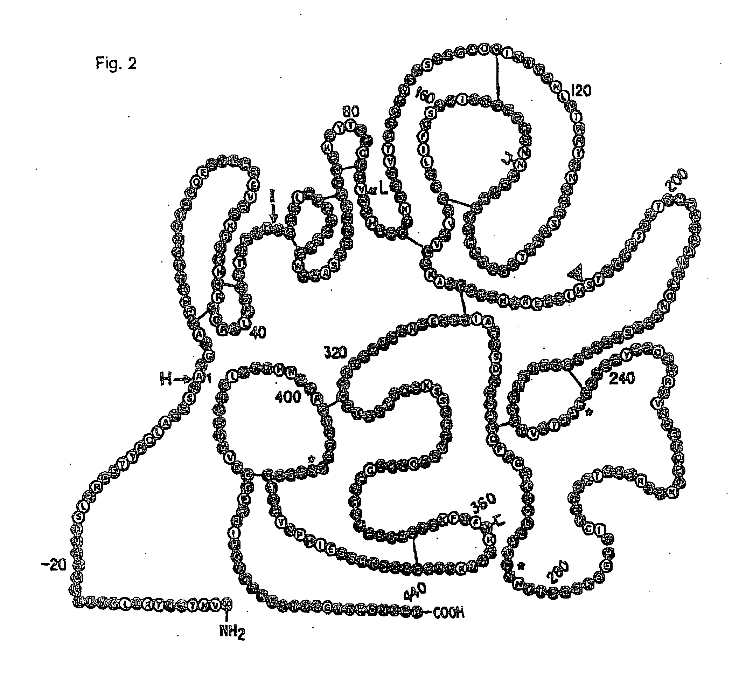

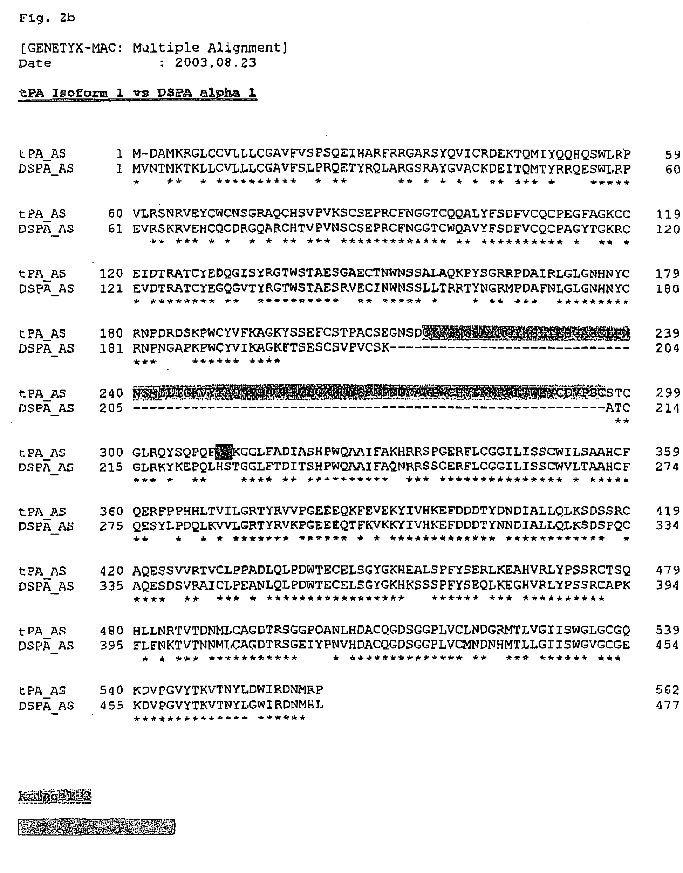

Eine schematische Darstellung der Struktur des DSPA ist als Fig. 2 beigefügt. Einen Vergleich der Aminosäuresequenz des t-PA mit derjenigen des DSPA zeigt Fig. 2b.

In der Vergangenheit wurde die Fibrin-Bindung des t-PA funktionell auf die Finger-Domaine und den Kringel-2 zurückgeführt (van Zonnenfeld AJ, Veerman H, Pannekoek H: (1986) Proc. Natl. Acad. Sci. USA., 83:4670-4674). Publikationen der jüngeren Zeit weisen jedoch auch der Proteinase Domaine P eine gewisse Relevanz bei (Bennett a.a.O). Die Funktion der einzelnen Domainen wurde auch von Bakker AHF, Jacoline E., Weening-Verhoeffet. D., Verheijen JH (The role of Lysyl-Binding site of tissue type Plasminogen activator in the interaction with a forming fibrin clod: Journal of Biological Chemistry 270, 21, 12355-12360. 1995) untersucht.

Bakker et al. stellten verschiedene Modifikationen des t-PA her, so zwei Deletionsmutanten, denen entweder der Kringel-2, die Finger-Domaine oder die epidermale Wachstumsfaktor-Domaine fehlten. Diese Mutationen wurden auch kombiniert und zum Teil zusätzlich mit einer Punktmutation im Kringel-2 versehen, nämlich mit der Substitution D236N. Diese gezielte Aminosäuresubstitution führt zu einem Austausch des Asp in der Position 236 zu Asparagin und damit zu einer Deletion der Lysin-Bindungsstelle (LBS in der K2-Domaine). Zur Bereitstellung der Mutation wird auf die genannte Veröffentlichung von Bakker et al. einschließlich der darin zitierten Referenzen ausdrücklich verwiesen.

Bakker et al. konnten in ihren Untersuchungen zeigen, daß die Besetzung der Lysin-Bindestelle in der K2-Domaine mit EACA (ε-Aminocapronsäure) die Bindung des nativen t-PA an Fibrin deutlich schwächte. Das gleiche gilt für die Modifikation der LBS durch die Substitution D236N, wobei diese Schwächung geringer ausfiel. Auch die Deletionsmutante, die lediglich aus der K2-Domaine und dem proteolytischen C-Terminus bestand, hat noch - wenn auch gering - an Fibrin gebunden. Nur die Mutation K2P mit deletierter LBS zeigte keinerlei Fibrin-Bindung mehr.

Diese Ergebnisse zeigen zwar zum einen die Bedeutung der F- und K2-Domainen so wie der LBS im K2. Sie zeigen jedoch auch, daß die Fibrininteraktion des t-PA nicht ausschließlich durch die F- und K2-Domaine vermittelt wird. Vielmehr tritt noch die Funktion der LBS in der K2-Domaine hinzu, die vermutlich zur Stabilisierung einer für die Fibrin-Bindung günstigen Konformation des t-PA verantwortlich ist.

Die Bedeutung der K2-Domaine einschließlich ihrer Lysin-Bindungsstelle wurde des weiteren durch Untersuchungen von Stewart RJ, Fredenburgh JG, Witz JI deutlich (Characterization of the interactions of plasminogen and tissue and vampire bat plasminogen activators with Fibrinogen, fibrin, and the complex of D-dimer noncovalently linked to fragment E: Journal of Biological Chemistry 273, 29, 18292-18299, 1998).

Stewart et al. haben in Bindungsstudien unter anderem die Affinitäten von t-PA und DSPA zu Fibrin und Fibrinogen untersucht. Dabei wurden die Affinitäten in Anwesenheit oder Abwesenheit des Lysinanalogs EACA untersucht, um die Bedeutung der Kringel abhängigen Interaktionen zu analysieren. Aus ihren Untersuchungen zogen sie den Schluß, daß DSPA aufgrund der fehlenden Lysin-Bindestellen im Kringel nicht an Fibrinogen binden kann. Sie messen daher der LSB am Kringel-2 des t-PA in Kombination mit der Finger-Domaine eine wesentliche Funktion für die Fibrinbindung des t-PA zu.

Die unter Beispiel 1 aufgeführten Versuchsergebnisse bestätigen die unterschiedlichen Affinitäten des DSPA α1 und des rekombinanten humanen t-PA für die Cofaktoren Amyloid β (1-42) sowie Fibrinogen und Fibrin. Dazu wurden die kinetischen Parameter kcat und Km sowie das Verhältnis kcat/Km für jede Plasminogen-Aktivator/CoFaktor-Kombination ermittelt. Bei der Abwesenheit eines Cofaktors war die Effizienz des DSPAα1 für die Plasminogen-Aktivierung etwa 100-fach niedriger als diejenige des t-PA, während sie bei der Anwesenheit von Fibrin beide gleichermaßen effizient waren. In Anwesenheit des Cofaktors Fibrinogen oder β-Amyloids war das DSPAα1 etwa 30-fach weniger effektiv als t-PA. Aus diesen Daten ergibt sich ein Verhältnis der Effizienz des Fibrins als Cofaktor zu den Cofaktoren Fibrinogen oder β-Amyloid bei einer physiologischen Plasminogen Konzentration von 2 µM von 480 für DSPAα1 und 16 für humanes t-PA.

Der erfindungsgemäß eingesetzte Plasminogen aktivierende Faktor ist - wie oben bereits ausgeführt - dadurch gekennzeichnet - daß er keine oder aber eine modifizierte Lysin-Bindungsstelle am Kringel-2 aufweist. In einer Ausführungsform ist der Kringel-2 deletiert. In einer weiteren Ausführungsform ist es jedoch auch möglich, den Kringel-2 zu erhalten und eine Substitution der Asparaginsäure durch Asparagin in der Position 236 vorzunehmen. Demnach ist es für die erfindungsgemäße Erfindung des modifizierten t-PA nicht entscheidend, daß die Kringel-Struktur fehlt, sondern lediglich, daß die Lysin-Bindungsstelle so modifiziert ist, daß eine die Aktivität des t-PA steigemde Interaktion mit einem Cofaktor nicht mehr möglich ist.

In einer weiteren vorteilhaften Ausführungsform kann der verwendete Plasminogen-Aktivator - insbesondere das modifizierte t-PA - durch die Deletion des Kringel-1 so modifiziert werden, daß eine Rezeptorbindung des t-PA nicht mehr möglich ist. Damit wird der Gewebe-Plasminogen-Aktivator nicht mehr in der nativen Weise über die Leber metabolisiert, so daß sich die *in vivo* Halbwertzeit verlängert (Rijken DC, Otter M, Kuiper J, von Berkel TJC: Receptor-mediated endocytosis of tissue-type plasminogen activator (t-PA) by liver cells. Thromb. Res. 1990; Supel. X: 63-71). Die Deletion des Kringel-1 ist von der Reteplase bekannt und beispielsweise von Martin U, Bader R, Böhm E, Kohnert U, von Möllendorf E, Fischer S. Sponder G. (BM 06.022: A Novel recombinant plasminogen activator. Cardiovascular drug reviews 1993;11: 299-311) beschrieben. Dazu werden Mutanten mit einer Deletion der Aminosäuren 4 - 176 eingesetzt. Durch die Verlängerung der Halbwertzeit ist eine Bolusapplikation des Plasminogen-Aktivators möglich.

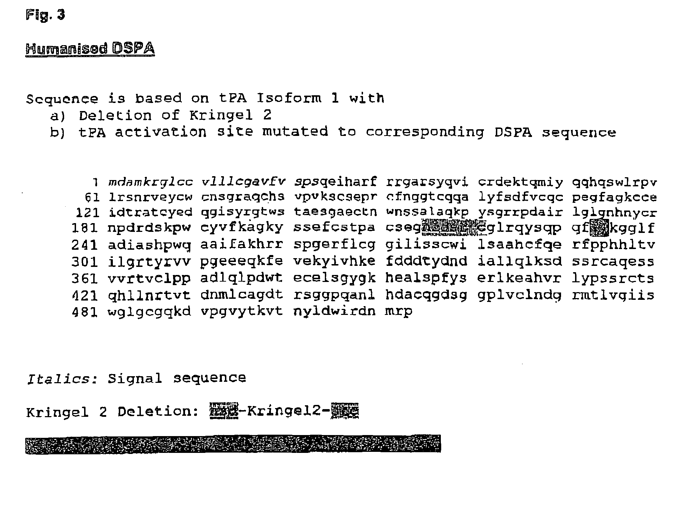

In einer bevorzugten Ausführungsform basiert der erfindungsgemäß einsetzbare Plasminogen-Aktivator auf einer der in den Figuren 3, 11 oder 13 gezeigten Aminosäuresequenzen. Dabei handelt es sich jeweils um ein modifiziertes t-PA mit einem deletierten Kringel 2 und einer strukturellen Änderung im Sequenzbereich der Aktivierungsspaltstelle des t-PA. Diese Änderung kann entweder in der Substitution lediglich der Aminosäuren R und I der Aktivierungsspaltstelle bestehen (z.B. durch HS; siehe Sequenz gemäß Fig. 3) oder auch noch benachbarte Aminosäuren betreffen (z.B. Substitution von FRIK durch LHST in der Sequenz gemäß Fig. 11). Die letztere Modifikation entspricht der Struktur des nativen DSPA an dieser Stelle.

Es wird erfindungsgemäß bevorzugt, einen Plasminogen Aktivator zu erzeugen und für die Schlaganfallbehandlung einzusetzen, der in besonders geeigneter Weise die Vorteile des nativen t-PA - nämlich vor allem eine niedrige Immunogenität bei der Verwendung in menschlichen Patienten - mit den Vorteilen des DSPA - nämlich die fehlende Neurotoxizität - verbindet. In einer Ausführungsform der Erfindung wird demnach die Deletion des Kringels 2 im t-PA so gewählt, daß ein Übergangsbereich zu der nachfolgenden Struktur geschaffen wird, der der vergleichbaren Struktur des DSPA entspricht. Der Übergangsbereich zwischen der verbleibenden Kringel 1 Domaine des modifizierten t-PA zu der nachfolgenden Cysteinbrücke wird demnach vorteilhafterweise durch die Sequenz SKAT gebildet. Die Sequenz SKAT findet sich im DSPA zwischen dem Kringel und der Cysteinbrücke. Diese vorteilhafte Struktur ist beispielsweise in der Sequenz gemäß Fig. 11 verwirklicht.

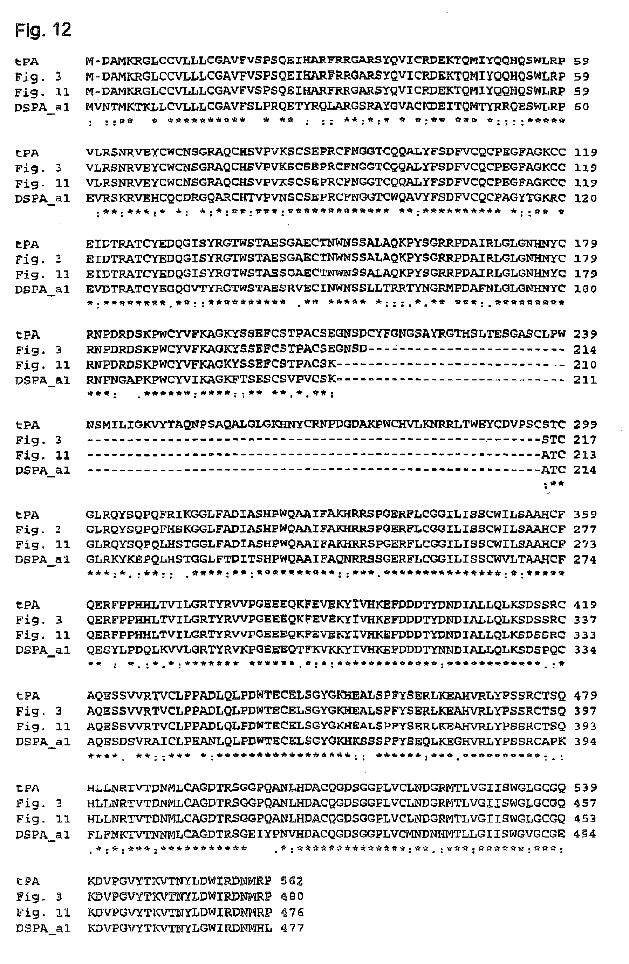

Einen Vergleich zwischen t-PA und den beiden geschilderten erfindungsgemäßen Ausführungsformen zeigt Fig. 12 (Multiple Sequence Alignment).

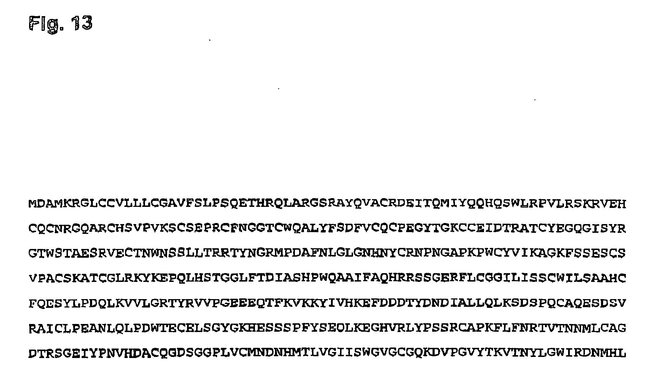

In einer weiteren erfindungsgemäßen Ausführungsform wird ein Plasminogen Aktivator gemäß der Figur 13 eingesetzt.

Es ist selbstverständlich auch möglich, erfindungsgemäß Proteine einzusetzen, die zu den in den Figuren 3, 11 und 13 dargestellten Aminosäuresequenzen homologe oder teil-identische Sequenzen aufweisen. Bevorzugt sind Homologien bzw. Identitäten von mindestens 70, bevorzugt zwischen 80 und 95%. Diese homologen oder identischen Proteine zeigen die Aktivität eines Plasminogen-aktivierenden Faktors (vorzugsweise dargestellt als Freigabe des pNA) und verursachen *in vitro* die Lyse eines Blutgerinnsels (siehe Beispiel 3).

Die erfindungsgemäß einsetzbaren Plasminogen-Aktivator können durch eine fehlende Finger- und epidermale Wachstumsfaktor-Domaine gekennzeichnet sein. Durch diese Deletion wird die Bindung an die Rezeptoren der Leber wesentlich vermindert und die Halbwertzeit nochmals verlängert (Larson GR, Timony GA, Horgan PG, Barone KM, Henson KS, Angus LB. Stoudemire JB: Protein engineering of novel plasminogen activators with increased thrombolytic potency in rabbits relative to activase. Journal of biological Chemistry 1991; 266:8156-8161; Smalling RW: Molecular biology of plasminogen activators: what are the clinical implications of drug design? Am J Cardiol 1996; 76 (suppl. 12): 2 - 7).

Neben Deletionsmutanten können die erfindungsgemäß eingesetzten Plasminogen-Aktivatoren auch durch gerichtete Mutagenese nur punktuell modifiziert werden, So ist beispielsweise aus der rt-PA-TNK (Tenecteplase) bekannt, daß drei Mutationen an der Bindungsstelle des Plasminogen-Aktivator Inhibitors einerseits und an der Bindungsstelle, über die t-PA an die Leberzellen bindet andererseits, zu einer Erhöhung der katalytischen Aktivität bei einer gleichzeitig verminderten PAI-Inaktivierbarkeit führen. Damit erhält die Tenecteplase eine 100-fach erhöhte katalytische Konversionskonstante Kcat/Km (s. dazu auch Paoni NF, Keyt BA, Refino CJ, Chow AM, Nguyen HV, Berleau LT, Badillo J, Pena LC, Brady K, Wurm FM, Ogez J, Bennett WF: A slow clearing fibrin-specific, PAI resistant variant of t-PA (T103N, KHRR 296-299 AAAA). Thromb Haemostas 1993; 70: 307 - 312 und Cannon CP. Love TW, McCabe CH, Kirshenbaum JM, Henry T, Sequira R, Schweifer M, Breed J. Cutler D, Tracy R. for the TIMI investigators. TNKtissue Plasminogen activators in myocardial infarction (TIMI) 10: Results of the initial patients in the TIMI 10 pilot - a phase 1, pharmacokinetics trial. Circulation 1995; 92 (suppl.):1-415).

### BEISPIEL 1:

1. Vergleichende Analyse der Effizienz von β-Amyloid (1 bis 42) Fibrinogen und Fibrin als Cofaktoren für die Plasminogen-Aktivierung durch DSPAα1 und rekombinanten humanen t-PA.

### Material und Methoden

### Die folgenden Substanzen wurden verwendet:

- DSPAα1 (bereitgestellt von Paion, Chargen-Nr. 2DSA01. 12. Dezember 2002, Probe 10). 10 mg davon wurden in 10 ml sterilem Wasser aufgelöst, um eine Endkonzentration von 1 mg/ml zu erhalten.
- Rekombinantes t-PA (Actilyse ® bereitgestellt von Paion, Chargen-Nr. 102572) 10 mg gelöst in 10 ml sterilem Wasser zu einer Endkonzentration von 1 mg/ml.
- Humanes Glu-Plasminogen gereinigt aus humanem Plasma (bereitgestellt von Paion) gelöst in PCLA-Puffer mit einer Konzentration von 25µM.
- Humanes Fibrinogen, im wesentlichen plasminogenfrei wurde erworben von Sigma (Katalog-Nr. F4883, Charge 12K7620). 25 mg wurden in 25 ml PCLA-Puffer aufgelöst zu einer Endkonzentration von 1 mg/ml.
- Humanes Thrombin (Sigma; Katalog-Nr. T7009. Charge 61 K7603) 100 U wurden in 10 ml PCLA-Puffer zu einer Konzentration von 10 U/ml aufgelöst.
- Farbreagenz Flavigen.pli (D-but-CHT-Lys-P-Nitroanolin-DHCL) von Biopool (Katalog-Nr. 101353, Charge 1512016). 100 µM wurden in 50 ml PLCR-Puffer zu einer Endkonzentration von 2 Milimolar aufgelöst.
- Amyloidprotein β (1 - 42) von Bachem (Katalog-Nr. H-1368, Charge 0535120). 4 mg wurden in 4 ml 0,1-prozentigen Ammoniumhydroxid zu einer Endkonzentration von 1 mg/ml aufgelöst.

Alle Reagenzien wurden in Aliquots geteilt und bei -20°C für nicht mehr als zwei Wochen gelagert.

PLC-Puffer (Jones 1990): 0,1 M NaCl.2H₂O (Acros Katalog-Nr. 20779); 0,03 M NaHCO₃ (Acros Katalog-Nr.21712); 4mM KCI (Fluka, Katalog-Nr, 60130); 1mM CaCl₂.2H₂ (Acros, Katalog-Nr, 207780); 1 mM Na₂HPO₄.2H₂O (Fluka, Katalog-Nr. 71638); 0,3 mM MgCl₂.6H₂0 (Acros, Katalog-Nr. 197530); 0,4 mM MgsO₄.7H₂0 (Fluka, Katalog-Nr. 63140); 20 mM HEPES (Applichem. Katalog-Nr. A1969); 0,01% Polysorbate 80 (Fluka, Katalog-Nr. 93781)

### Plasminogen-Aktivierungsassay:

Der Assay für die Plasminogen-Aktivierung wurde auf Mikrotiterplatten beim Gesamtvolumen von 0,15 ml durchgeführt wie von Bringmann et al. (a. a. O.) beschrieben. Die Regenzien wurden wie folgt zugefügt:

50 µl Plasminogen (0 - 24 µM); 15 µl Cofaktor (1 mg/ml); 10 µl Plasminogen-Aktivator 7,5 nM und 75 µl Flavigen (2 mM).

Die Endkonzentration war wie folgt:

Plasminogen-Aktivator: (t-PA oder DSPAα1): 0,5 nM: Glu-Plasminogen; 0,0625, 0,125, 0.25, 0,5, 1, 2, 4 und 8 µM; FlavigenPli: 1mM und Cofaktoren: 100µg/ml.

Es wurden die Cofaktoren Amyloid β (1 - 42), Fibrinogen und Fibrin mit der Kontrolle ohne Cofaktor verglichen. Für die Kontrolle enthielt der Reaktionsansatz weitere 0,13 Units/ml humanen Thrombins.

Direkt nach dem Hinzufügen wurde die Mikrotiterplatte in einen *molecular device ThermoMax microplate reader* bei 25°C eingebracht.

Ab Zeitpunkt t = 0 wurde gerührt.

Nach regelmäßigen Intervallen wurde die optische Dichte bei 405 nm und 490 nm gemessen. Die optischen Dichten bei 490 nm wurde subtrahiert von den optischen Dichten bei 405 nm, um die Unterschiede aufgrund der Flüssigkeitsbewegung zu eliminieren. Alle Versuche wurden in drei Wiederholungen mit jeder Plasminogenkonzentration und Plasminogenaktivatorkonzentration durchgeführt.

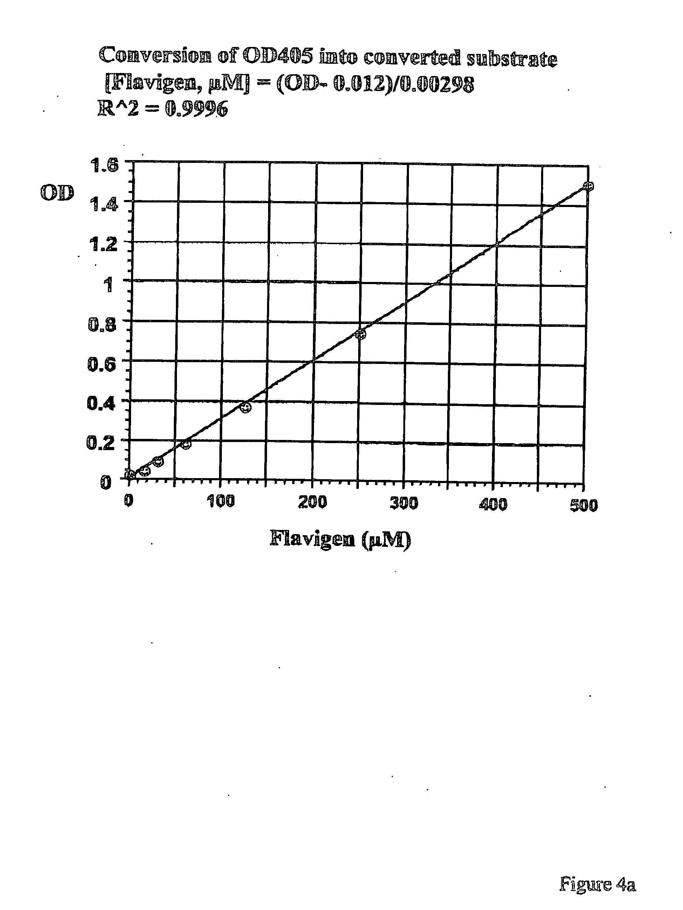

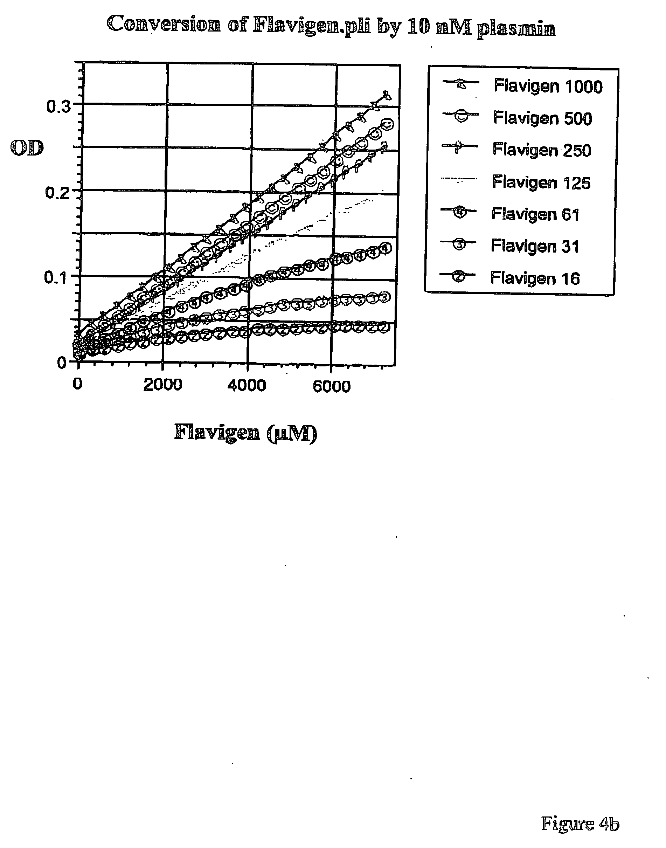

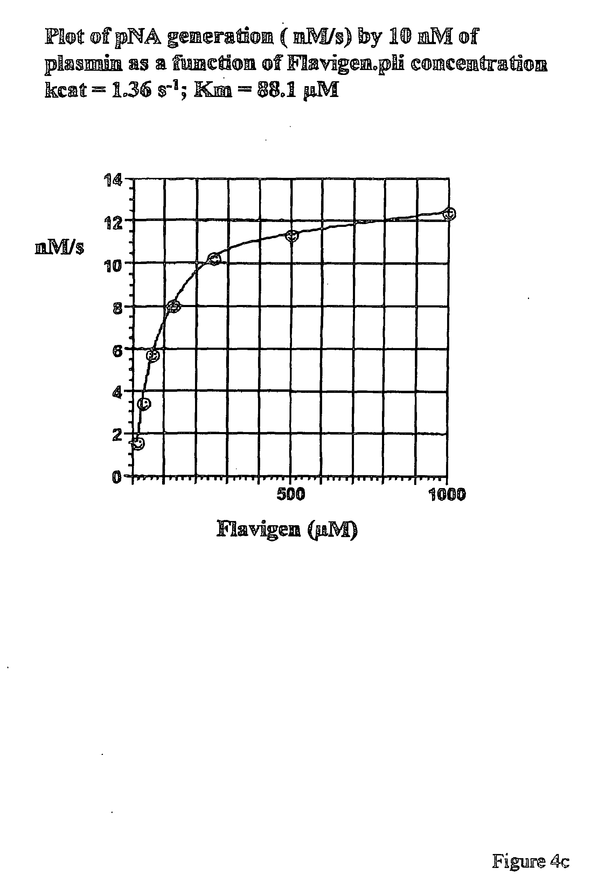

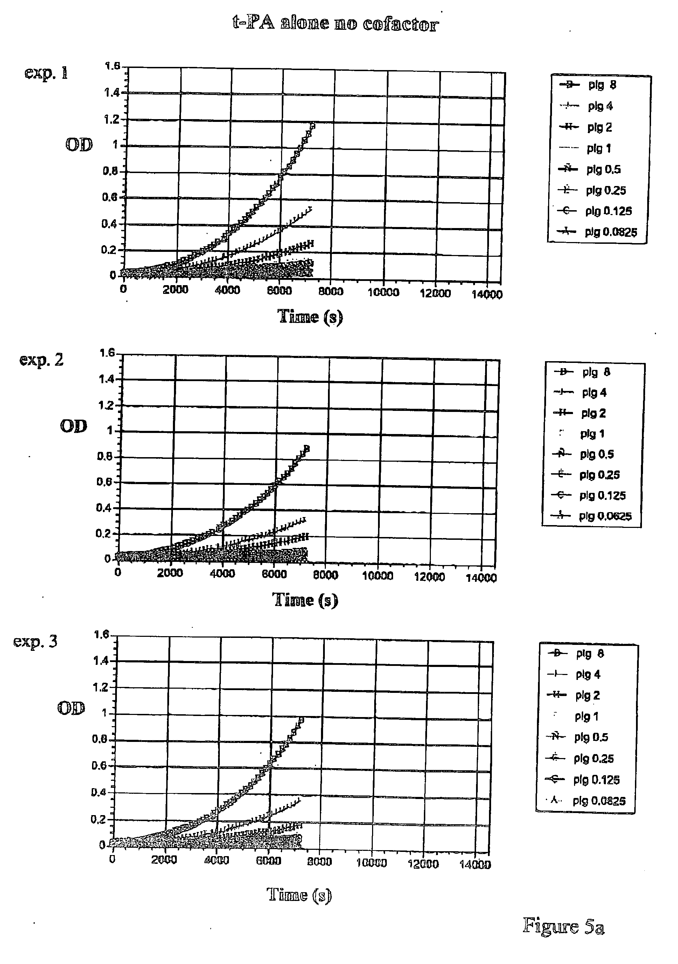

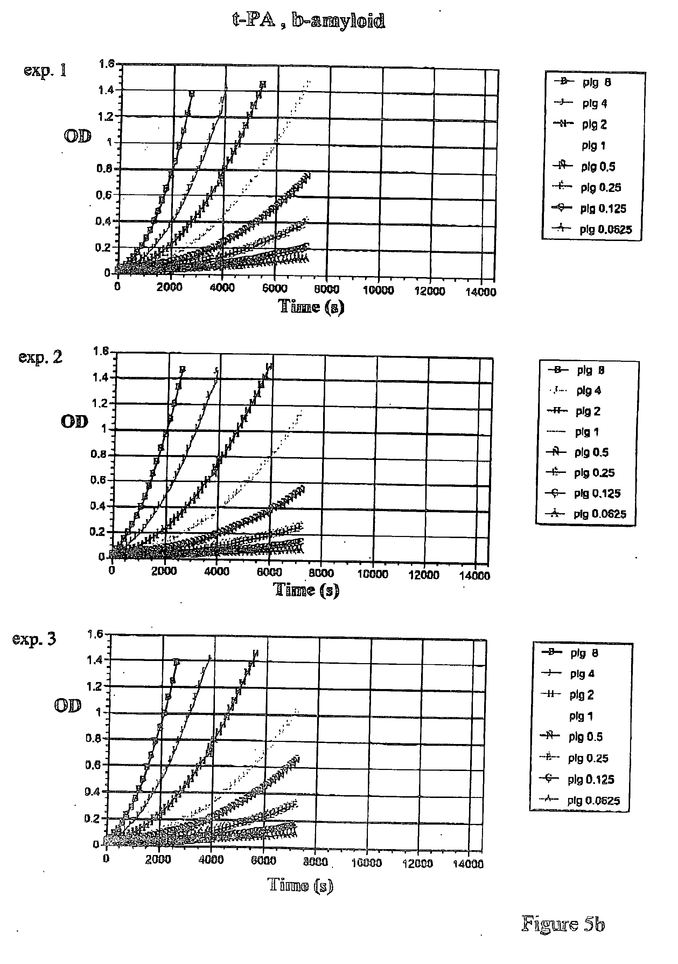

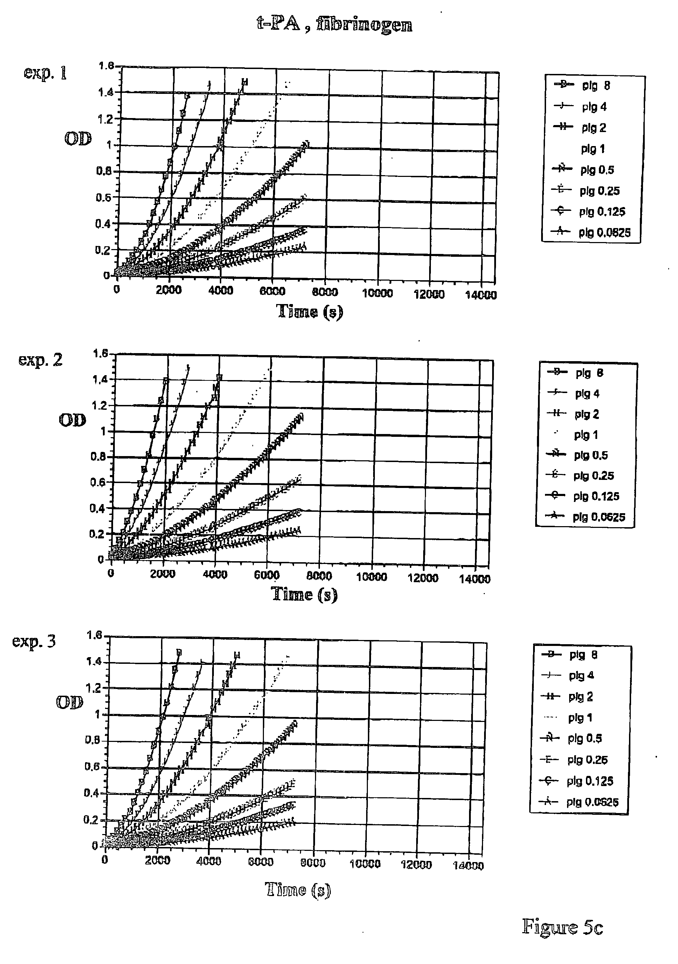

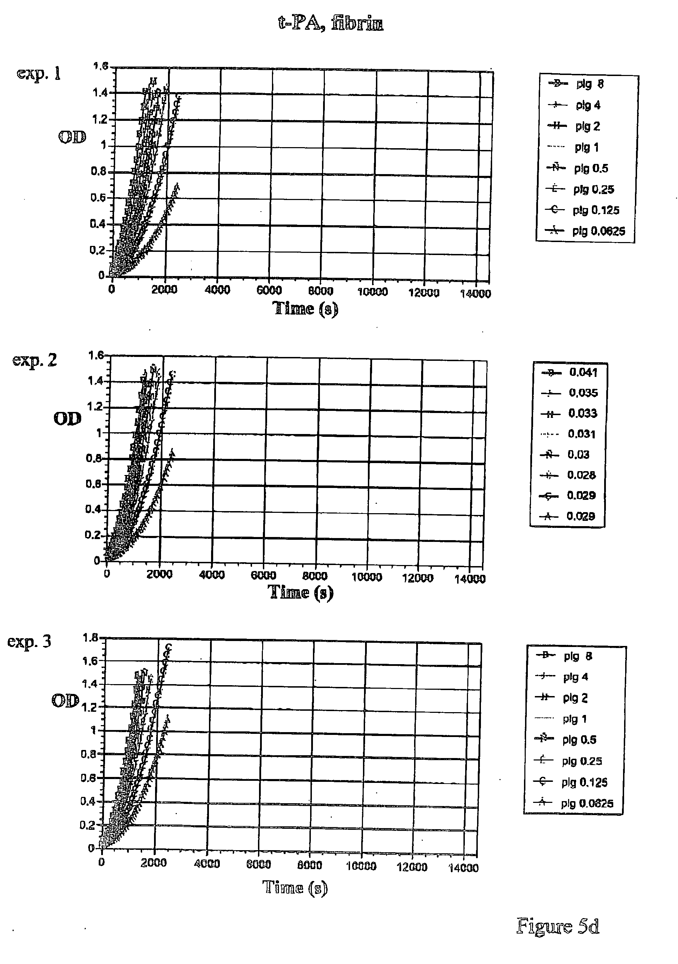

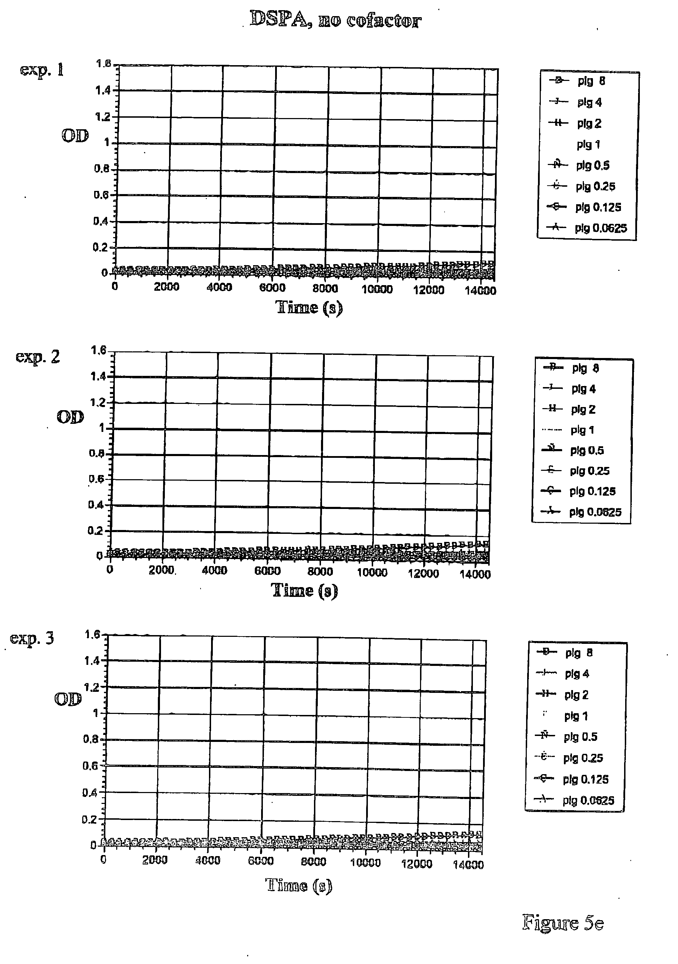

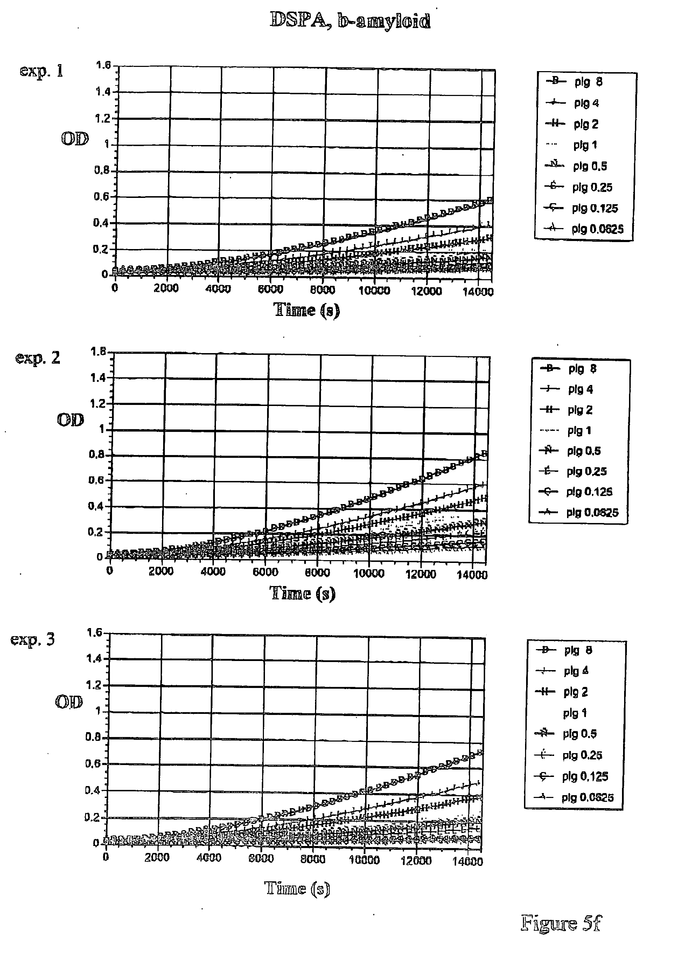

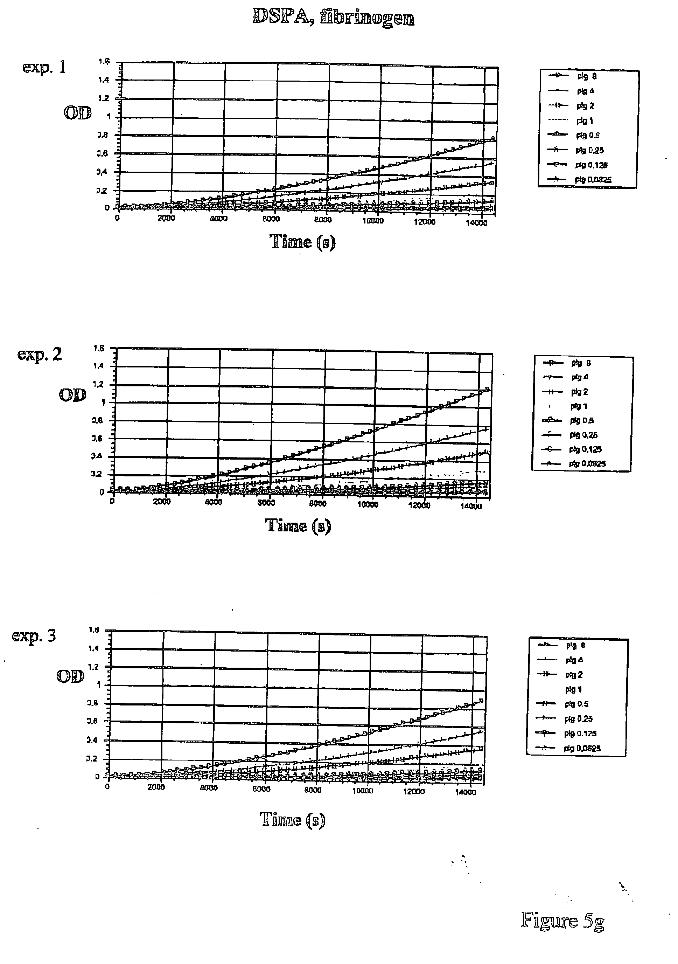

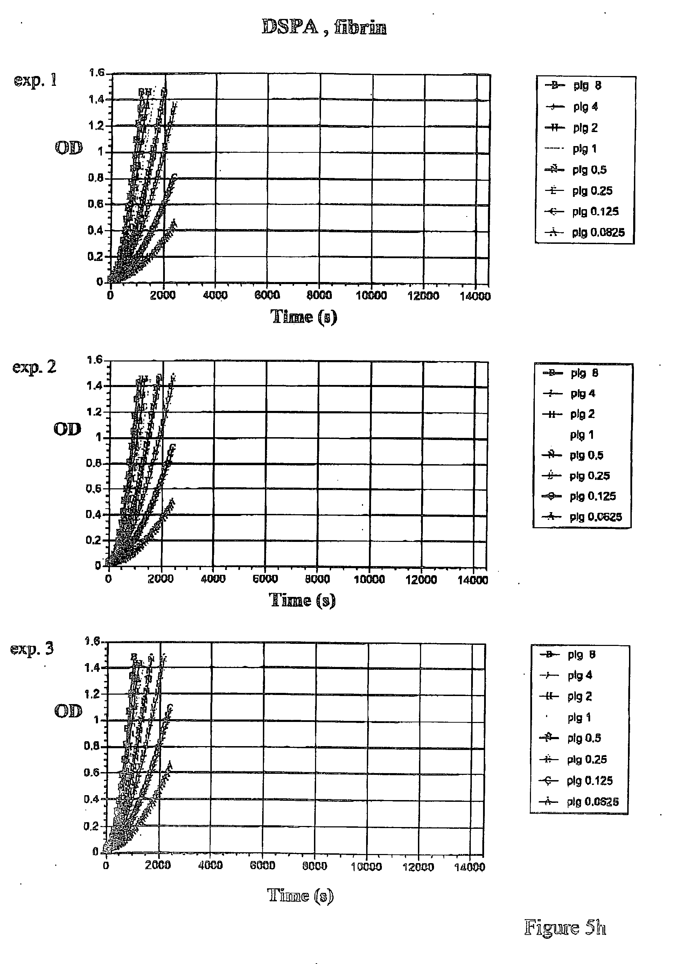

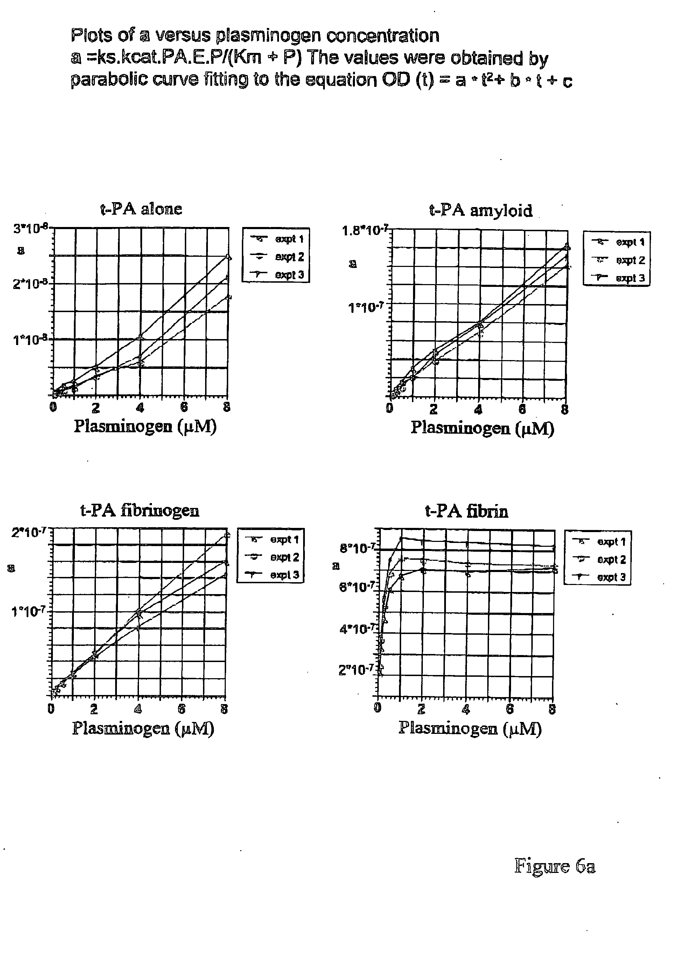

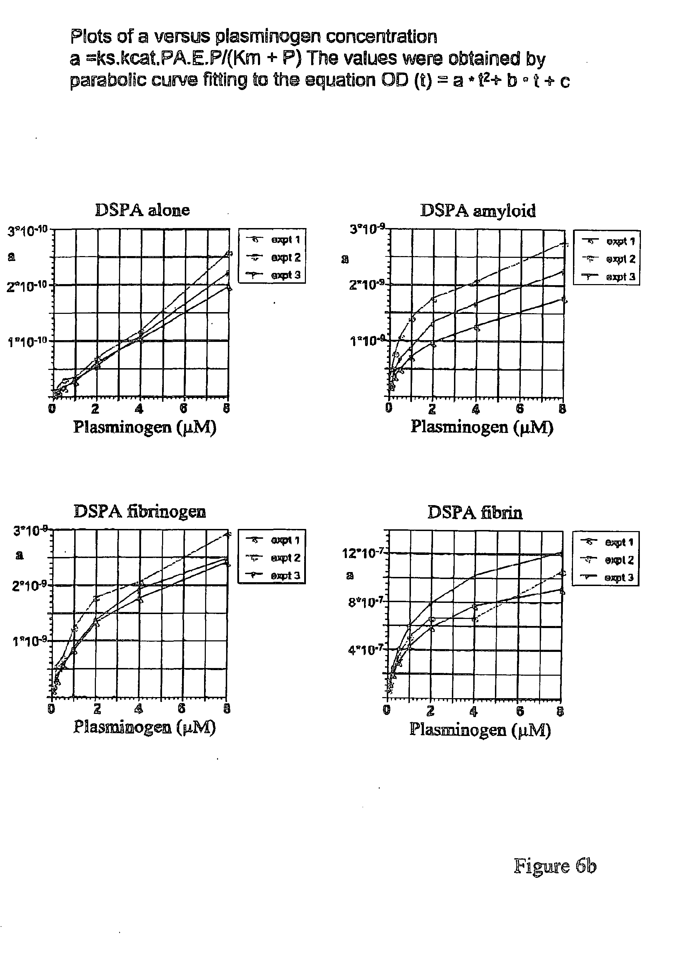

Es wurden die Km- und kcat-Werte ermittelt (Fig. 4 - 6).

### Figuren 4

a) Bestimmung der molaren Extinktion des pNA.
b) Konversion des Flavigen.pli durch 10 nM Plasmin als Funktion der Flavigen.pil-Konzentration (in µM).
c) Michaelis-Menten plot der Konversion von Flavigen durch 10 nM Plasmin. Die Anfangsraten aus Fig. 4b wurden umgesetzt in pNA/s.

### Figuren 5

Kurven der optischen Dichte gegen die Zeit von vier verschiedenen Kombinationen von Plasminogen-Aktivatoren und Cofaktoren. Dazu waren Plasminogenkonzentrationen von 8; 4; 2; 1; 0,5; 0,25; 0,125 und 0,0625 µM eingesetzt.
a) t-PA ohne Cofaktor
b) t-PA mit Amyloid β auf (1 - 42) als Cofaktor
c) t-PA mit Fibrinogen als Cofaktor
d) t-PA mit Fibrin als Cofaktor
e) DSPAα1 ohne Cofaktor
f) DSPAα1 mit Amyloid β (1 - 42) als Cofaktor
g) DSPAα1 mit Fibrinogen als Cofaktor
h) DSPAα1 mit Fibrin als Cofaktor

### Figuren 6

Plot von a {= ks.kcat.PA.E.P./(Km + P)} gegen die Plasminogenkonzentration
a) Plots für -PA ohne Cofaktor oder Amyloid β, Fibrinogen oder Fibrin als Cofaktor
b) Plots für DSPAα111.38 ohne Cofaktoren

Ergebnisse:

**Tabelle 1**

| | kcat (s⁻¹) | Km(µM) | kcat/Km |
|---|---|---|---|
| t-PA ctrl | n.d. | n.d | 1272 ± 224 |
| t-PA amyl | n.d. | n.d | 9307 ± 579 |
| t-PA fbg | n.d. | n.d | 10204 ± 1546 |
| t-PA fibrin | 0.351 ± 0.35 | 0.178 ± 0.017 | 1990000 ± 327'000 |
| DSPA ctrl | n.d. | n.d | 12.5 ± 0.9 |
| DSPA fbg | 1.649 ± 0.110e-3 | 2.465 ± 0.426 | 690 ± 181 |
| DSPA amyl | 0.957 ± 0.199e-3 | 0.853 ± 0.316 | 1304 ± 785 |
| DSPA fibrin | 0.532 ± 0.136 | 1.098 ± 0.382 | 506000 ± 120'000 |

Tabelle 1: Werte für kcat/Km für die Aktivierung von Plasminogen durch rekombinantes humanes T-PA oder DSPAα1 mit β-Amyloid (1 bis 42), Fibrinogen oder Fibrin als Cofaktoren.

Die Ergebnisse sind angegeben als Mittel ± Standardabweichung von drei unabhängigen Versuchen.

Die genauen Werte für kcat und Km konnten nicht bestimmt werden, da die Km-Werte höher als die maximale Plasminogenkonzentration (8 µM) lagen, die einsetzbar waren.

Die Ableitung der kinetischen Konstanten allein ist nicht ausreichend, um die Effizienz der verschiedenen Cofaktoren zu vergleichen, da die Reaktionsraten bei Plasminogenkonzentrationen nahe oder unterhalb der Km-Werte abhängig sind von der Plasminogenkonzentration. Daher werden - unter Heranziehung der Michaelis-Menten-Kinetik - die Reaktionsraten bei physiologischen Plasmaplasminogenkonzentrationen bei 2 µM berechnet. Davon ausgehend werden die relativen Effizienten der verschiedenen Cofaktoren für die Plasminogen-Aktivierung durch t-PA und DSPAα1 (Tabelle 2) ermittelt.

**Tabelle 2**

| cofactor | DSPAa1 | t-PA |
|---|---|---|
| none | 1 | 101.8 ± 17.9 |
| DSPA amyloid | 27.3 ± 7.7 | 744.6 ± 23.6 |
| DSPA fbg | 29.8 ± 5.0 | 816,3 ± 123.7 |
| DSPA fibrin | 13560 ± 2360 | 12880 ± 1360 |

Relative Raten der Plasminogenaktivierung bei der physiologischen Plasminogenkonzentration von 2 µM berechnet ausgehend von den kinetischen Parametern der Tabelle 1. Die Kombination der DSPA-Kontrolle ist 1.

### BEISPIEL 2:

Die Prionen-Erkrankungen, die spongiformen Encephalopatien sind fatale neurodegenerative Erscheinungen, die durch eine Akkumulation der Abnormalen Isoform des Prionproteins (PrP^{5C}) in Amyloid Ablagerungen charakterisiert sind. Das normale Prionenprotein (PrP^{C}) ist in vielen Geweben exprimiert worden, vor allem im Gehirn, in dem es konzentriert in den Synapsen vorliegt. Der Mechanismus der Konformationsänderung von PrP^{c} zu PrP^{sc} ist noch wenig verstanden ebenso wie der Mechanismus der über die Bildung der PrP^{sc} zu Neurodegeneration führt. Ein möglicher Mechanismus könnte in der Beeinflussung des PrP^{sc} bei der Plasminogen-Aktivierung durch t-PA liegen.

Wir haben bereits unter Verwendung eines rekombinanten Proteins gezeigt, daß PrP die t-PA katalysierte Plasminogen-Aktivierung um das 300fache steigern kann (Ellis V, Daniels M. Misra R, Brown DR (2000): Plasminogen activation is stimulated by prion protein and regulated in a copper-dependent manner. Biochemistry 41:6891-6896).

Die Fähigkeit des PrP zur Stimulierung der Plasminogen-Aktivierung war abhängig von der PrP Konformation, die durch die Gegenwart oder Abwesenheit von Cu² 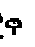 beeinflußt war. Die letztere Form enthält eine große Anzahl von β-Strukturen die PrP^{sc} Isoform entspricht und die Plasminogen-Aktivierung stimuliert. Diese Daten stimmten überein mit früheren Beobachtungen der Plasminugen-Bindung an PrP^{5c}, das von scrapie-infizierten Mäusegehirnen isoliert wurde, jedoch nicht von PrP^{c} als normalen Gehirnzellen (Fischer M. Roeckl C, Rarizek P, Schwarz HP Aguzzi A (2000): Binding of diseaseassociated prion protein to plasminogen. Nature 408: 479-483).

Weiterhin haben unsere Experimente gezeigt. daß der Mechanismus der PrP stimulierten Plasminogen-Aktivierung auch die Bindung von t-PA apo-PrP (d.h. PrP ohne Cu²⁺) umfaßt. Die Strukturen, die für diese Bindung erforderlich sind. sind im Einzelnen noch nicht evaluiert. Die Bindung ist jedoch spezifisch, da sie durch DFP-inaktiviertes t-PA verhindert wurde.

Weitere Methoden:
Die PrP Ansätze die in diesen Untersuchungen verwendet wurden, wurden jeweils aus rekombinanten murinen PrP aus E.coli abgeleitet. Im Anschluß an die Reinigung der His markierten PrP faltet sich das Protein wieder in der Abwesenheit oder Gegenwart von Kupferchlorid, um entweder ein holo-PrP oder ein apo-PrP zu bilden. Aggregierte Formen dieser Proteine werden jeweils durch schnelles Lösen in Wasser erzeugt. Das Protein wurde durch Zentrifugation gesammelt. Das Material ist resistent gegen Proteinase-K Verdauung. Eine mutante Form von Δ51-90, das die Cu²⁺ Bindungsstelle nicht umfaßt, ist in gleicher Weise hergestellt worden. Die Wirkung dieser verschiedenen Formen PrP auf die Plasminogen-Aktivierung durch DSPA wurde untersucht durch die Inkubation von PrP (0-100 µg/m) mit Lys-Plasmionogen (25 nm) und DSPA (0,25 nm). Die Plasminogen-Aktivierung wird ermittelt durch die Hydrolyse der plasminspezifischen Farbstoffs H-d-Val-Leu-Lys-7-amido-4-methylcoumarin bei 37°C unter Verwendung der des SPECTRAmax Gemini lourescence microplate reader. Die Kontrollen dieser Experimente enthalten sc-t-PA, als positive Kontrolle für den Effekt des PrP auf die Plasminogen-Aktivierung, und Firbinfiragmente als positive Kontrolle für die Stimulierung der t-PA sowie der DSPA Aktivität.

Die Spezifität der Plasminogenbindung wurde bestimmt durch Kompetition mit DFP-inaktiviertem t-Pa. Als kompetitiver Hemmstoff wurde auch das Lysinanalogon EACA eingesetzt.

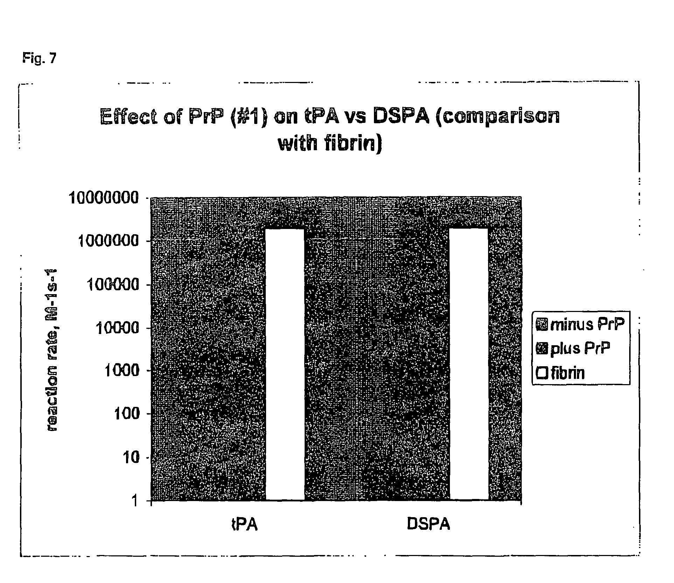

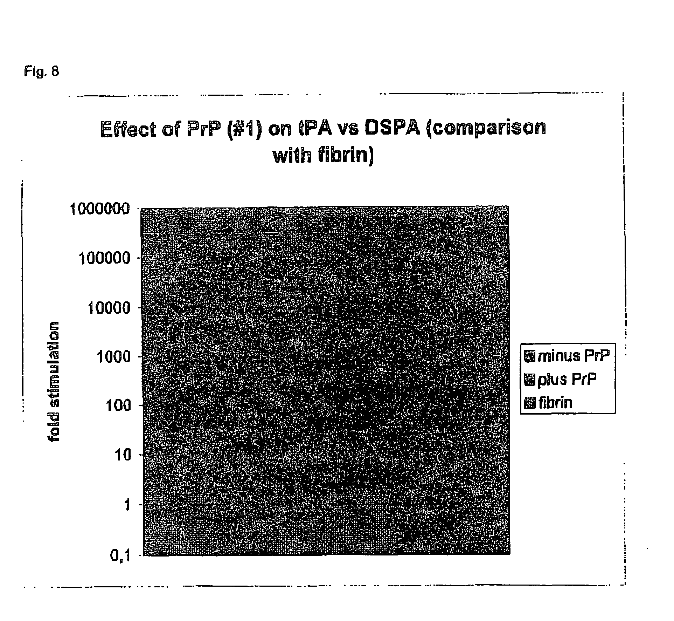

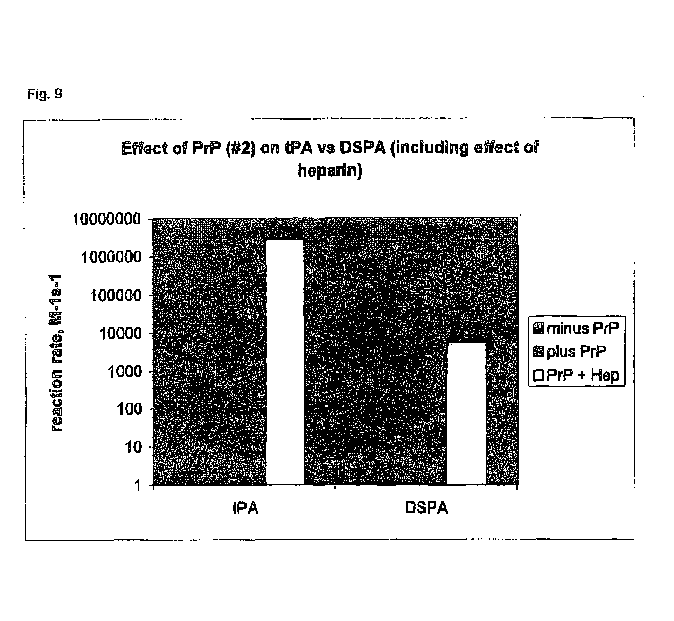

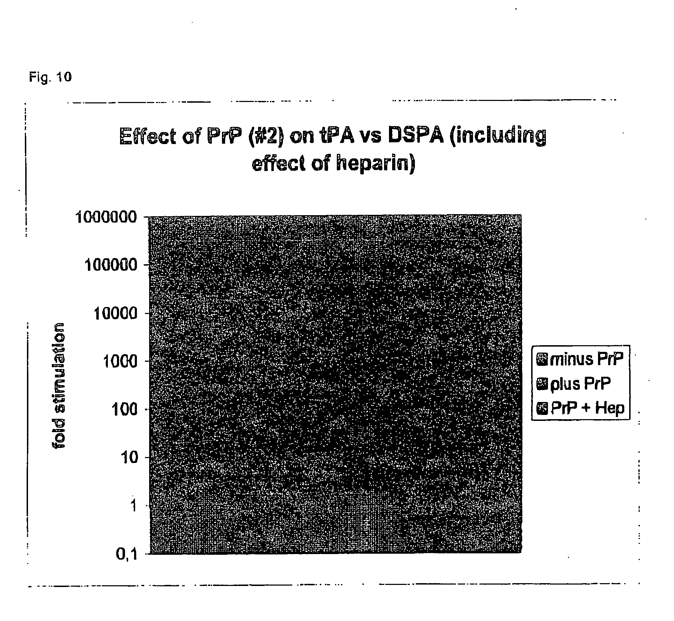

Ergebnisse:
Die Ergebnisse der Versuche sind in der Tabelle 3 und den Fig. 7 bis 10 dargestellt.

Eine inhaltliche Aussage wird insbesondere durch die Fig. 7 verdeutlicht. Diese macht klar, daß PrP spezifisch nur t-PA aktiviert, nicht jedoch DSPA. Des weiteren wurde deutlich, daß die Lysin-Bindestelle Kringel 2 als wesentlicher Unterschied zwischen DSPA und t-PA involviert sein muß. Tabelle 3

### Reaction rates, M-1s-1

| | | **minus PrP** | **plus PrP** | **PrP + Hep** | **fibrin** |
|---|---|---|---|---|---|
| PrP preparation #1 | | | | | |
| | **tPA** | 7200 | 1800000 | | 2000000 |
| | **DSPA** | 15,3 | 9,77 | | 2000000 |
| PrP preparation #2 | | | | | |
| | **tPA** | 7020 | 624000 | 2912000 | |
| | **DSPA** | 15,75 | 162,75 | 5565 | |
| **Fold-stimulation** | | | | | |

| | | **minus PrP** | **plus PrP** | **PrP + Hep** | **fibrin** |
|---|---|---|---|---|---|
| PrP preparation #1 | | | | | |
| | **tPA** | 1 | 250 | | 277,7778 |
| | **DSPA** | 1 | 0,638562 | | 130719 |
| PrP preparation #2 | | | | | |
| | **tPA** | 1 | | 88,88889 | 414,8148 |
| | **DSPA** | 1 | | 10,33333 | 353,3333 |

### BEISPIEL 3:

### a. Herstellung und Reinigung von humanisiertem DSPA (humDSPA)(Aminosäuresequenz gemäß Fig. 11)

Mit dem Bac-to-Bac System (Invitrogen) wurde rekombinante humanDSPA-Baculovirus-DNA hergestellt und die gereinigte Virus-DNA in Sf9-Insektenzellen transfiziert. Der produzierte rekombinante Baculovirus wurde mit Hilfe von Sf-9 Zellen amplifiziert, Die Expression von humDSAP erfolgte mit High Five-Insektenzellen in serumfreien Medium in Suspensionskultur. Der Zellkulturüberstand mit dem rekombinanten Protein wurde bis zur weiteren Aufarbeitung bei -20°C eingefroren.

Der Zellkulturüberstand wurde langsam bei RT auf einem Schüttler aufgetaut und 1 Stunde bei 4000xg abzentrifugiert. Die Proteinlösung (1.51) wurde mit 50 mM Ammoniumacetat auf pH 6.0 equilibriert, an eine SP-Sepharose XL (Amersham) lonenaustauschersäule (350 ml Bettvolumen) geladen und mit 5 Säulenvolumina 50 mM Ammoniumacetatlösung gewaschen. Das gebundene Protein wurde in einem Gradienten von 0-100% 1M Ammoniumacetat pH 6 über 10 Säulenvolumina eluiert.

HumDSPA haltige Fraktionen wurden über Westernblot identifiziert, nach Proteinbestimmung wurden solche Fraktionen einem Aktivitätstest unterzogen, bei -80°C eingefroren und später lyophilisiert.

Im Unterschied zum Standard Aktivitätsassay wurden in diesem Experiment 50µl Eluat (=humDSPA haltige Fraktion), 50 µl 0,2M Tris pH 8.0 und 100 µl 2 mM S-2288 in PBS eingesetzt.

### b. DSPA Aktivitätsassay

Beim eingesetzten Aktivitätsassay handelt es sich um eine Bestimmung der Geschwindigkeit, mit der ein Plasminogenaktivator das farblose Substrat S-2288 in ein farbiges Produkt umsetzt. Der Assay stellt ein Standardverfahren zur Bestimmung der proteolytischen Aktivität von Plasminogen aktivierenden Faktoren dar. Zur Darstellung der Aktivität wird die Freigabe eines Chromogens (p-Nitroanilin, pNA) durch die Testsubstanz ermittelt. Dazu bietet die Firma Chromogenix das Chromogen S-2288 an.

Die Reaktionsgeschwindigkeit wird dabei nicht direkt bestimmt (z.B. in Katal), sondern mit einem Standard verglichen, der als 100% Aktivität definiert ist.

Die Messung der Reaktionsgeschwindigkeit wurde in einem Puffer durchgeführt, der folgende Zusammensetzung hatte: 25mM Tris-HCl pH8 / 0,1% Albumin / 100mM NaCl/1.1 mM Glycine / 1.2 mM Mannitol / 2.5mM S-2288

Die Messung wurde in einer 96-Loch Platte mit Leerwerten. Standards und Proben mit unterschiedlichen Proteinkonzentrationen durchgeführt.

Die zeitliche Zunahme der Absorption bei 405 nm wurde mit Hilfe eines Photometers aufgezeichnet. Die Steigung des linearen Teils der Kurve wurde mit Hilfe von Excel bestimmt. Dies gibt die Aktivität wieder.

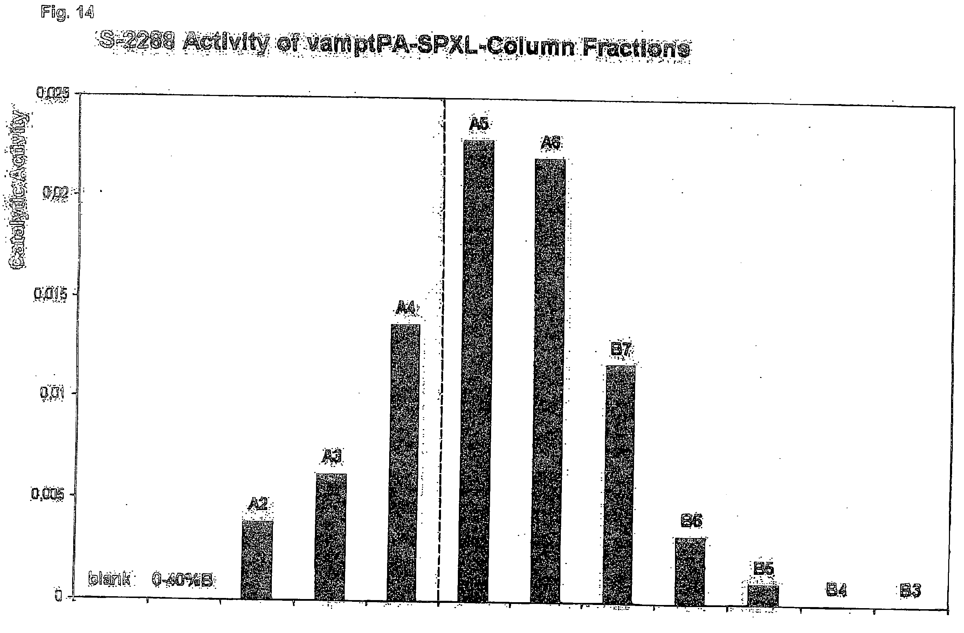

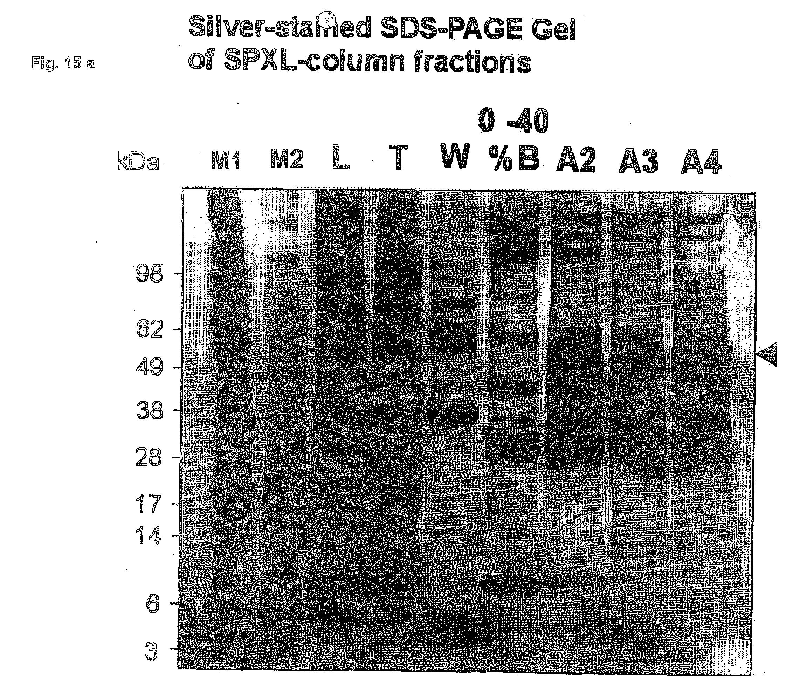

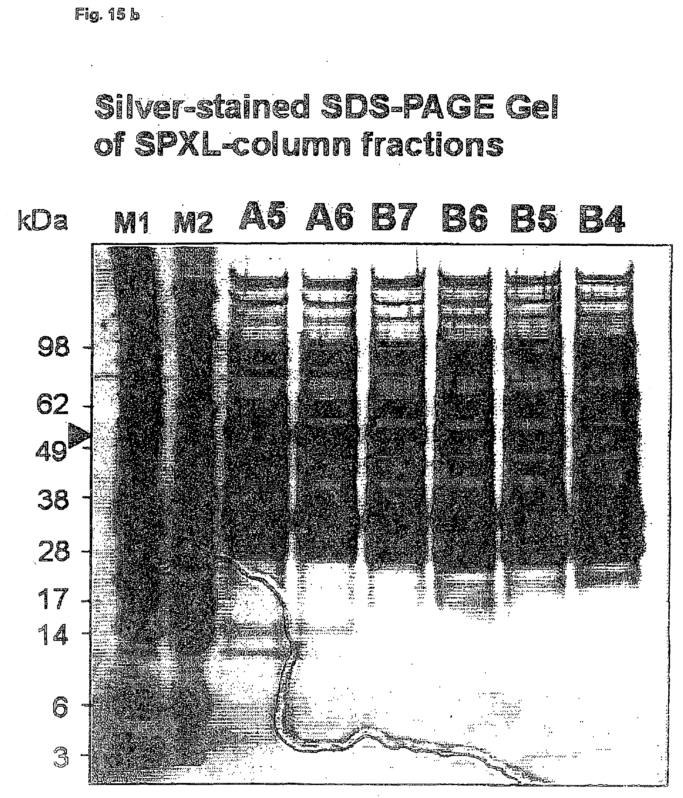

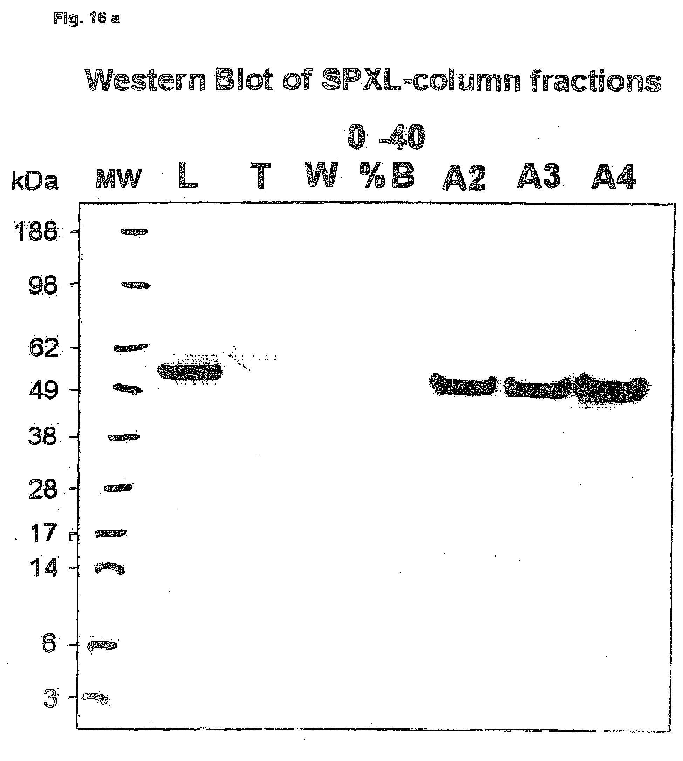

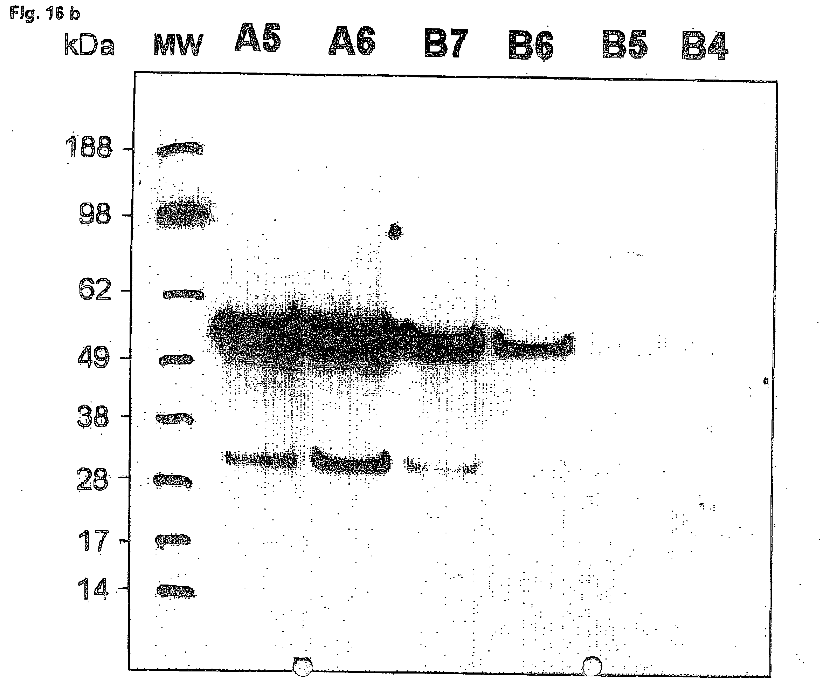

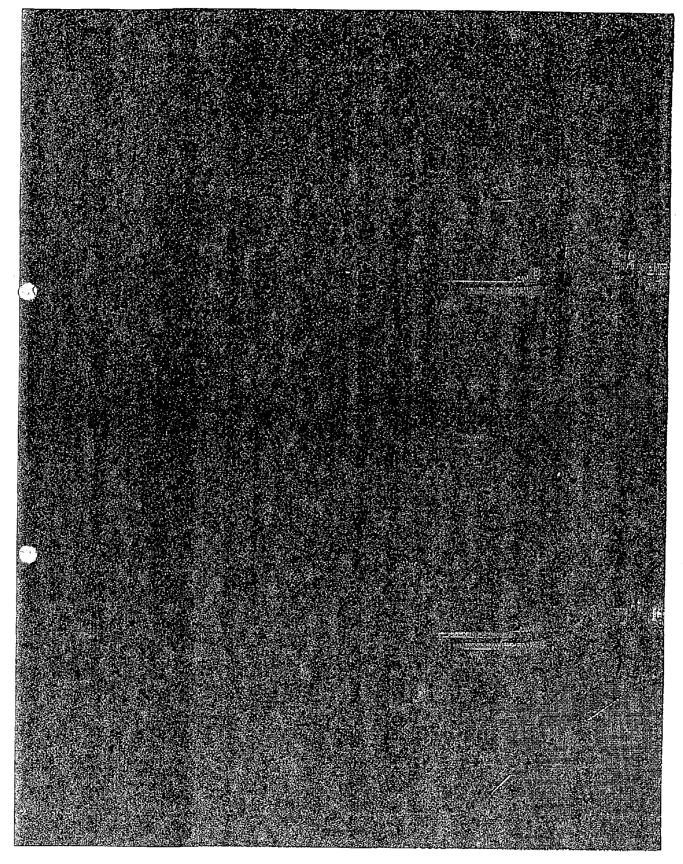

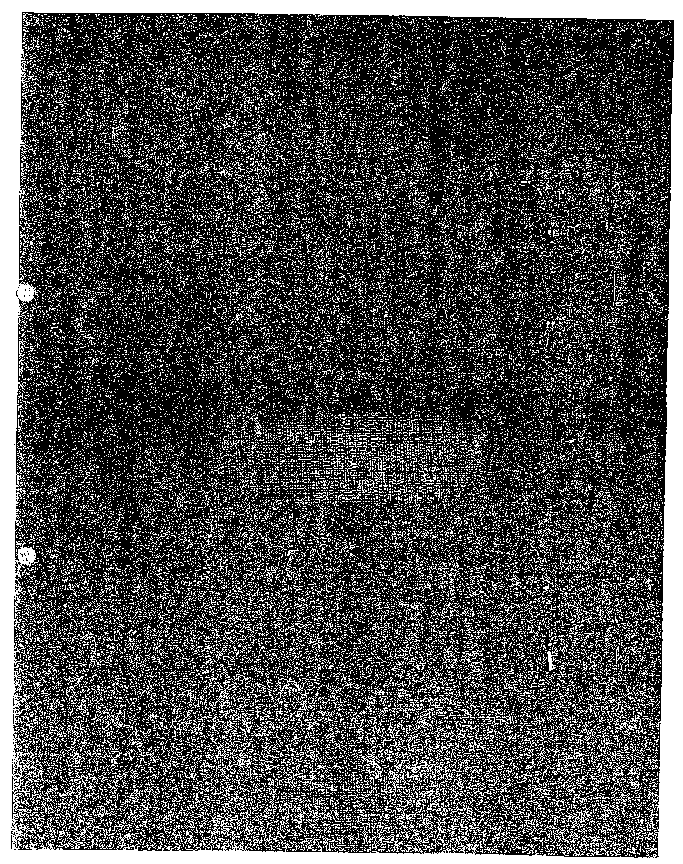

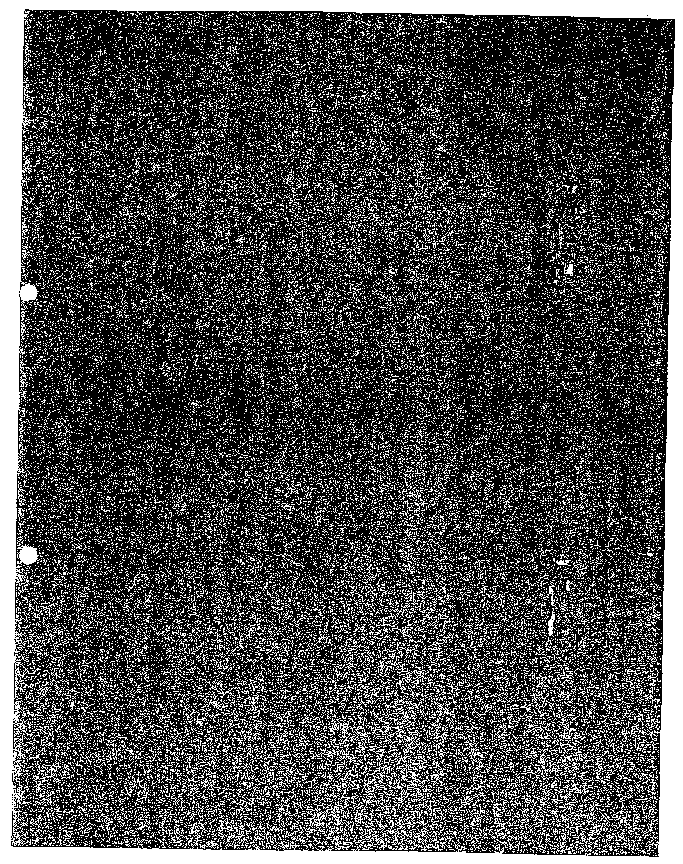

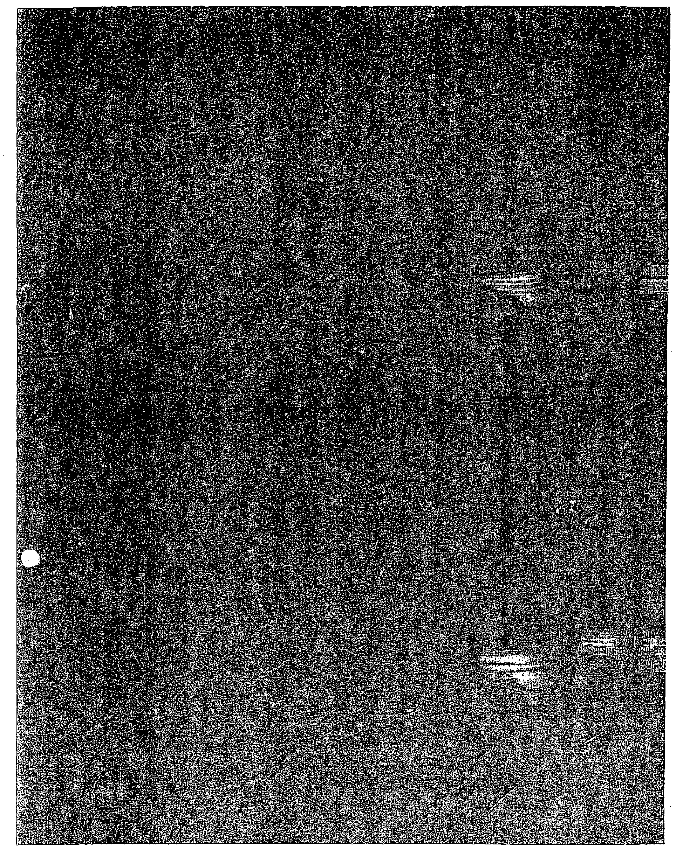

Die Aktivität des Fraktionen (ausgedrückt in S-2288 Aktivität) zeigt die Fig. 14. Die silbergefärbten Gele sind in den Fig. 15 a und 15 b zu sehen. Fig. 16a und 16b zeigen die Westem-Biots. Das im Western Blot dedektierte Protein entspricht dem mit der Pfeilspitze markierten Protein im silbergefärbten Gel.

### [L= Load; T _Durchfluß; W=Wash; 0-40%B, A2, A3... Fraktionen]

### BEISPIEL 4:

### Clot-Lyse (thrombolytische Aktivität)

Der Rückstand der humaDSPA-haltigen Fraktion B6 wurde in 15 ml PBS aufgelöst und 15 min bei 4000xg abzentrifugiert. Diese Fraktion wurde ausgewählt, da sie die größte Reinheit zeigte. Ein Aliquot wurde zur Aktivitätsbestimmung abgenommen und benutzt. Die katalytische Aktivität dieser Probe war ungefähr mit der katalytischen Aktivität von 5 µg/ml Activase vergleichbar.

Die verwendeten Blutgerinnsel entstammen einer normalen Blutabnahme 24 h früher und sind aus jeweils 2 ml Blut entstanden, das in Polypropylen-Röhrchen überführt wurde und unter natürlichen Bedingungen gerann. Die Lyse wurde in PBS durchgeführt.

Die humaDSPA-haltige Fraktion B6 (von SPXL-Sepharose Capture Schritt) wurde zunächst lyophilisiert. Der Rückstand wurde in 15 ml PBS aufgelöst und 15 min/4000g abzentrifugiert. Davon wurde vor Zugabe des Blutgerinnsels eine Probe für eine S-2288 Aktivitätsbstimmung abgenommen. Die katalytische Aktivität dieser Probe entsprach im wesentlichen der katalytischen Aktivität von 5 µg/ml Activase.

Die Figuren 17a bis 17d zeigen die Ergebnisse der Clot-Lyse im zeitlichen Verlauf von 0h, 3h, 4h und 24h nach Zusatz des humDSPA. Der linke Versuchsansatz zeigt jeweils die Kontrolle. In den rechten Ansätzen ist PBS mit humaDSPA in der oben beschriebenen Menge enthalten.

Die Blutzellen fallen langsam aus dem sich auflösenden Gerinnsel heraus. Nach 24 h bleibt eine Art Netz übrig, das bereits nach 4 h erkennbar wird. In dem Ansatz nach 4 h wurden abgelösten Blutzellen mit einer Spritze abgesaugt, damit die Struktur des verbliebenen Gerinnsels sichtbar wurde.

Es gilt zu beachten, daß sich die Fibrinolyse verlangsamt, wenn das Gerinnsel nicht mehr im eigenen Lysat hängt. Daher ist nach 24 h überhaupt noch ein Gerinnsel vorhanden.

Eine Quantifizierung dieser Reaktion ist nicht möglich. Im Vergleich zu nativem DSPA zeigt das humDSPA aber eine deutliche Aktivitätssteigerung, da für die Auflösung eines Gerinnsels derselben Größe durch DSPA mindestens die 4fache Menge DSPA verwendet werden muß.

## Patentansprüche

1. Verwendung eines Plasminogen-Aktivators mit einer gegenüber dem nativen t-PA verringerten Lysin-Bindungskpazität zur Behandlung thrombotischer Erkrankungen, insbesondere Schlaganfall.

2. Verwendung nach Anspruch 1, gekenntzeichnet durch eine Modifikation des Plasminogen-Aktivators am Kringel 2 oder dazu funktionell oder strukturell homologer Domainen.

3. Verwendung nach Anspruch 2, gekenntzeichnet durch eine Deletion des Kringels 2 oder dazu strukturell oder funktionell homologer Domainen.

4. Verwendung nach Anspruch 2, **gekennzeichnet durch** eine Modifikation der Lysin-Bindungsstellen des Kringels 2 oder dazu strukturell oder funktionell homologer Domainen.

5. Verwendung nach Anspruch 4, **gekennzeichnet durch** die Substitution D236N oder dazu homologer Aminosäuren.

6. Verwendung nach einem der vorherigen Ansprüche, **dadurch gekennzeichnet, daß** Plasminogen-Aktivator eine mindestens 75%-ige Homologie zu t-PA aufweist.

7. Verwendung nach Anspruch 6, **dadurch gekennzeichnet, daß** die t-PA-Aktivierungsstelle durch Deletion oder Substitution modifiziert ist, so daß keine protolytische Spaltung statt findet.

8. Verwendung nach Anspruch 7, **gekennzeichnet durch** eine Modifikation der Aminosäureposition R15-I16.

9. Verwendung nach Anspruch 8, **dadurch gekennzeichnet, daß** die Aktivierungsstelle eine Sequenz LHST aufweist.

10. Verwendung nach einem der vorherigen Ansprüche, dadurch gekennzeichnen, daß der Plasminogen-Aktivator im Übergangsbereich zwischen dem kringel und der Cysteinbrücke einen Aminosäuresequenzabschnitt mit SKAT aufweist.

11. Plasminogen-Aktivator umfassend eine Aminosäuresequenz gemäß einer der Figuren 3, 11 oder 13.

12. Plasminogen-Aktivator **gekennzeichnet durch** eine Identität von mindestens 70 %, vorzugsweise eine Identität von 80 bis 90%-, besonders bevorzugt eine Identität von 95 % zu einem Plasminogen-Aktivator nach Anspruch 10.

13. Plasminogen-Aktivator nach einem der Ansprüche 10 bis 12 **gekennzeichnet durch** eine gegenüber dem nativen DSPA erhöhten thrombolytischen Aktivität.
